Europäisches Patentamt

European Patent Office

Office européen des brevets

(19)

(11) Veröffentlichungsnummer : **0 371 339 B1**

# (12) EUROPÄISCHE PATENTSCHRIFT

(45) Veröffentlichungstag der Patentschrift :
**03.03.93 Patentblatt 93/09**

(51) Int. Cl.⁵ : **A61K 7/08,** A61K 7/50,
C11D 1/37

(21) Anmeldenummer : **89121215.1**

(22) Anmeldetag : **16.11.89**

(54) Oberflächenaktive Mischungen.

Verbunden mit 89912959.7/0445159
(europäische
Anmeldenummer/Veröffentlichungsnummer)
durch Entscheidung vom 23.09.91.

(30) Priorität : **25.11.88 DE 3839769**

(43) Veröffentlichungstag der Anmeldung :
**06.06.90 Patentblatt 90/23**

(45) Bekanntmachung des Hinweises auf die
Patenterteilung :
**03.03.93 Patentblatt 93/09**

(84) Benannte Vertragsstaaten :
**AT BE CH DE ES FR GB IT LI NL SE**

(56) Entgegenhaltungen :
**EP-A- 0 199 131**

(56) Entgegenhaltungen :
**EP-A- 0 209 910
CHEMICAL ABSTRACTS, Band 108, Nr. 8, 22.
Februar 1988, Seite 397, Zusammenfassung
Nr. 62262u, Columbus, Ohio, US; & JP-A-62 190
298 (JOHNSON, S.C., AND SON, INC.) 20-
08-1987**

(73) Patentinhaber : **Henkel
Kommanditgesellschaft auf Aktien
Henkelstrasse 67
W-4000 Düsseldorf 13 (DE)**

(72) Erfinder : **Müller, Reinhard, Dr.
Senliserstrasse 32
W-4018 Langenfeld (DE)**
Erfinder : **Wisotzki, Klaus-Dieter, Dr.
Neuendickstrasse 9
W-4132 Kamp-Lintfort (DE)**
Erfinder : **Giede, Karl
Schlehenweg 12
W-4010 Hilden (DE)**

EP 0 371 339 B1

Anmerkung : Innerhalb von neun Monaten nach der Bekanntmachung des Hinweises auf die Erteilung
des europäischen Patents kann jedermann beim Europäischen Patentamt gegen das erteilte europäische Patent Einspruch einlegen. Der Einspruch ist schriftlich einzureichen und zu begründen. Er gilt
erst als eingelegt, wenn die Einspruchsgebühr entrichtet worden ist (Art. 99(1) Europäisches Patentübereinkommen).

## Beschreibung

Die Erfindung betrifft oberflächenaktive Mischungen mit geringer Hautbelastung.

Um die Reinigungswirkung wäßriger Zubereitungen zu verstärken, enthalten diese üblicherweise oberflächenaktive Verbindungen. Allerdings ist diese gesteigerte Reinigungswirkung in der Regel mit einer erhöhten Hautbelastung verbunden. Dies gilt besonders für die wichtige Klasse der Aniontenside.

Zwar ist diese erhöhte Hautbelastung für übliche Verwendungen unproblematisch, jedoch sollte im Bereich der Körperreinigungsmittel eine erhöhte Hautbelastung vermieden werden. Dies gilt insbesondere für Personen mit empfindlicher oder vorgeschädigter Haut, für Kleinkinder und Babys. Aber auch bei Produkten, die für eine häufige Anwendung konzipiert sind, zur Reinigung des Intimbereiches dienen oder mit Schleimhäuten in Berührung kommen, ist eine gute Hautverträglichkeit wichtig.

Es besteht daher ein Bedarf an Körperreinigungsmitteln, die sich bei ausreichender Reinigungsleistung und zufriedenstellenden anderen Eigenschaften durch eine deutlich geringere Hautbelastung auszeichnen.

Es ist bekannt, daß durch Einsatz von Magnesiumionen bei Aniontensiden wie den Fettalkoholsulfaten und Ethersulfaten Produkte mit verbesserter Hautverträglichkeit erhalten werden können.

Die europäischen Patentanmeldungen 199 131 und 209 910 betreffen Ester von Hydroxycarbonsäuren wie beispielsweise der Zitronensäure und der Weinsäure. Durch Ersatz eines Teiles der üblichen Aniontenside durch solche Ester werden Aniontensidlösungen mit verbesserter Hautverträglichkeit erhalten.

Gleichfalls bekannt ist, daß sich ampholytische und nichtionogene Tenside im Vergleich zu Aniontensiden häufig durch eine verbesserte Hautverträglichkeit auszeichnen. Diese Tensidklassen können jedoch für viele Anwendungsbereiche nicht als Ersatz für Aniontenside verwendet werden, da sie in entscheidenden Punkten, wie beispielsweise der Reinigungswirkung, deren Eigenschaften nicht erreichen. Auch die für Shampoos vom Konsumenten gewünschte Schaumbildung ist bei ausschließlicher oder überwiegender Verwendung von Amphotensiden in der Regel nicht gewährleistet.

Aus einem in "Ärztliche Kosmetologie 13, 39-45 (1983)" veröffentlichten Artikel ist zu entnehmen, daß die Hautverträglichkeit von Anion-/Amphotensid-Mischungen in Einzelfällen über der der reinen Aniontenside liegt. Somit läßt sich die Hautverträglichkeit von Tensidlösungen dadurch steigern, daß die Aniontenside teilweise durch Amphotenside ersetzt werden, es müssen jedoch hinsichtlich wichtiger Eigenschaften wie Reinigungswirkung und Schaumbildung Nachteile in Kauf genommen werden.

Es bestand daher die Aufgabe, Reinigungsmittel zu finden, die sich sowohl durch eine hohe Reinigungsleistung und Schaumbildung, als auch durch eine deutlich geringere Hautbelastung auszeichnen.

Es wurde nun überraschenderweise gefunden, daß bei bestimmten Kombinationen von, innerhalb der Klasse der Aniontenside vergleichsweise wenig hautbelastenden, Aniontensiden eine synergistische Zunahme der Hautverträglichkeit auftritt. Auf Basis dieser Tensidkombinationen ist somit die Formulierung von Reinigungsmitteln möglich, die die Vorteile von Aniontensiden wie hohe Reinigungsleistung und große Schaumbildung mit einer gesteigerten Hautverträglichkeit verbinden.

Gegenstand der Erfindung sind somit oberflächenaktive wäßrige Mischungen enthaltend Fettalkoholethersulfate sowie mindestens ein weiteres Aniontensid aus der Gruppe der Fettalkoholethertartrate und Fettalkoholethercitrate, dadurch gekennzeichnet, daß (a) die Mischungen 0,01-3 Gew.-% an Erdalkalimetall-Ionen, bezogen auf die gesamte Mischung, enthalten und (b) die Fettalkoholethersulfate und die genannten weiteren Aniontenside in einem Mengenverhältnis von 70:30 bis 1:99 vorliegen.

Unter Fettalkoholethersulfaten sind die Sulfate der Anlagerungsprodukte von Ethylenoxid (EO) und/oder Propylenoxid (PO) an gesättigte und/oder ungesättigte, lineare und/oder verzweigte Fettalkohole zu verstehen, die nach bekannten Verfahren zugänglich sind. Die den Fettalkoholethersulfaten zugrunde liegenden Fettalkohole können reine Verbindungen sein. Es ist jedoch üblicherweise bevorzugt, Mischungen aus verschiedenen Fettalkoholen zu verwenden, die aus nativen Rohstoffen wie Fetten und Ölen erhalten werden. Diese Fettalkohole können mit den Alkylenoxiden beispielsweise unter Druck und bei Anwesenheit von Katalysatoren zu Fettalkoholalkoxylaten umgesetzt werden. Es ist dem Fachmann bekannt, daß bei Alkoxylierungsreaktionen wie beispielsweise der Anlagerung von n mol Ethylenoxid an 1 mol Fettalkohol nach den bekannten Ethoxylierungsverfahren kein einheitliches Addukt, sondern ein Gemisch aus Restmengen freien Fettalkohols und einer Reihe homologer (oligomerer) Anlagerungsprodukte von 1, 2, 3, ... n, n+1, n+2 ...usw. Molekülen Ethylenoxid je Molekül Fettalkohol erhalten wird. Der mittlere Ethoxylierungsgrad (n) wird dabei definiert durch die Ausgangsmengen an Fettalkohol und Ethylenoxid. Die Verteilungskurve des Homologengemisches weist in der Regel ein Maximum im Bereich zwischen n-3 und n+3 auf. Nähere Informationen zu diesen Punkten können beispielsweise der Zeitschrift Soap/Cosmetics/Chemical Specialities, Heft Januar 1988, S. 34, entnommen werden. Durch Umsetzung der Fettalkoholalkoxylate mit beispielsweise Schwefeltrioxid und anschließender Neutralisierung mit Alkalimetall-, Erdalkalimetall- oder Ammoniumhydroxiden- werden schließlich die gewünschten Fettalkoholethersulfate erhalten.

Es ist bevorzugt, solche Fettalkoholethersulfate einzusetzen, deren lineare oder verzweigte Alkyl- oder Alkenylreste 8-22 C-Atome enthalten. Gleichfalls ist bevorzugt, Fettalkoholethersulfate zu verwenden, die einen mittleren Alkoxylierungsgrad von 2-15, insbesondere 3-10, aufweisen. Dabei ist die Verwendung von ethoxylierten Produkten besonders bevorzugt. Es ist im Rahmen der Erfindung bevorzugt, Fettalkoholethersulfate einzusetzen, die bereits als Gegenionen die erfindungsgemäß benötigten Mengen an Erdalkalimetall-Ionen enthalten.

Erfindungsgemäß einzusetzende Fettalkoholethertartrate sind Monoester der Weinsäure mit Alkoholen, die Anlagerungsprodukte von Ethylenoxid und/oder Propylenoxid an Fettalkohole darstellen. Diese Fettalkohole können gesättigt und/oder ungesättigt, linear und/oder verzweigt sein. Bevorzugt ist es, Fettalkohole mit 8-22 C-Atomen einzusetzen. Gleichfalls bevorzugt sind Alkoxylierungsgrade von 2-15, insbesondere von 3-10. Hinsichtlich der Bedeutung des Alkoxylierungsgrades wird auf die bei der Beschreibung der Fettalkoholethersulfate gemachten Ausführungen verwiesen. Die monoveresterte Weinsäure kann sowohl direkt als auch in Form ihrer Salze, die durch Umsetzung der nicht veresterten Carboxylgruppe mit entsprechenden Basen zugänglich sind, verwendet werden. Bevorzugte Salze sind die Alkalimetall-, Erdalkalimetall- und Ammoniumsalze. Herstellungvorschriften für diese Verbindungen können der europäischen Patentanmeldung 209 910 entnommen werden.

Erfindungsgemäß einzusetzende Fettalkoholethercitrate sind Mono-und/oder Diester der Zitronensäure mit Alkoholen, die Anlagerungsprodukte von Ethylenoxid und/oder Propylenoxid an Fettalkohole darstellen. Diese Fettalkohole können gesättigt und/oder ungesättigt, linear und/oder verzweigt sein. Bevorzugt ist es, Fettalkohole mit 8-22 C-Atomen einzusetzen. Gleichfalls bevorzugt sind Alkoxylierungsgrade von 2-15, insbesondere von 3-10. Hinsichtlich der Bedeutung des Alkoxylierungsgrades wird ebenfalls auf die Ausführungen bei der Beschreibung der Fettalkoholethersulfate verwiesen. Die mono- und/oder diveresterte Zitronensäure kann sowohl direkt als auch in Form ihrer Salze, die durch Umsetzung der nicht veresterten Carboxylgruppe oder -gruppen mit entsprechenden Basen zugänglich sind, verwendet werden. Bevorzugte Salze sind die Alkalimetall-, Erdalkalimetall- und Ammoniumsalze. Herstellungsvorschriften für diese Verbindungen können der europäischen Patentanmeldung 199 131 entnommen werden.

Sowohl hinsichtlich der Fettalkoholethertartrate als auch hinsichtlich der Fettalkoholethercitrate sind die ethoxylierten Verbindungen bevorzugt.

Eine besonders gute Hautverträglichkeit wurde bei Mischungen gefunden, die die Fettalkoholethersulfate und die genannten weiteren Aniontenside in einem Mengenverhältnis von 60:40 bis 10:90 enthalten.

Besonders gute Eigenschaften zeigen Mischungen, die eine Kombination von Fettalkoholethersulfaten und Fettalkoholethertartraten enthalten. Ein ganz besonders bevorzugtes Fettalkoholethertartrat ist dabei das Natriumlauryl-(7 EO)-tartrat.

Am Rahmen der erfindungsgemäßen Lehre sind solche Mischungen bevorzugt, die 0,05-1 Gew.-%, insbesondere 0,05-0,5 Gew.-%, an Erdalkalimetall-Ionen enthalten. Dem Magnesium-Ion kommt dabei besondere Bedeutung zu.

Die oberflächenaktiven Mischungen enthalten insgesamt 2-50 Gew.-% an Aniontensiden. Ein Gehalt von 5-30 Gew.-% ist bevorzugt.

Die erfindungsgemäßen oberflächenaktiven Mischungen können neben den Aniontensiden zusätzlich 0,5-20 Gew.-%, insbesondere 1-10 Gew.-%, an ampholytischen und/oder zwitterionischen Tensiden enthalten.

Unter ampholytischen Tensiden werden solche oberflächenaktiven Verbindungen verstanden, die außer einer $C_8$-$C_{18}$-Alkyl- oder -Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarkosine, 2-Alkylaminopropionsäuren und Alkylaminoessigsäuren mit jeweils etwa 8 bis 18 C-Atomen in der Alkylgruppe.

Als zwitterionische Tenside werden solche oberflächenaktiven Verbindungen bezeichnet, die im Molekül mindestens eine quartäre Ammoniumgruppe und mindestens eine -COO($^-$)- oder -$SO_3$($^-$)-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Die hautschonenden Eigenschaften der erfindungsgemäßen Mittel kommen besonders dann zur Geltung, wenn diese so formuliert werden, daß sie einen pH-Wert in der Nähe des Neutralpunktes aufweisen. Mittel mit pH-Werten im Bereich von 5,5 - 7,5, insbesondere von 6,5 - 7,5, sind daher bevorzugt.

Die erfindungsgemäßen Mittel können in einer Vielzahl von Konsumentenprodukten wie Haarpflegemitteln, Haarshampoos, Körperpflege-Lotionen, Duschbädern und Produkten für die Säuglingspflege Verwendung finden.

Diese Produkte enthalten neben den beschriebenen Tensidkombination die üblichen Bestandteile wie

EP 0 371 339 B1

Emulgatoren, Ölkomponenten, Fette und Wachse, Lösungsvermittler, Verdickungsmittel, Überfettungsmittel, biogene Wirkstoffe, Filmbildner, Duftstoffe, Farbstoffe, Perlglanzmittel, Schaumstabilisatoren, Konservierungsmittel und pH-Regulatoren.

Als Emulgatoren können die in kosmetischen Zubereitungen üblichen Substanzen wie z. B. Fettsäurepartialglyceride, Fettsäure-sorbitan-partialester und deren Ethoxylate, Seifen, Fettalkoholsulfate, Polyol-Fettsäureester, Fettalkoholpolyglykolether, Lanolin, Wollfettalkohole und Alkylphosphate verwendet werden.

Übliche Ölkomponenten sind Substanzen wie Paraffinöl, Pflanzenöle, Fettsäureester, Squalan und 2-Octyldodecanol, während als Fette und Wachse beispielsweise Walrat, Bienenwachs, Montanwachs, Paraffin und Cetyl-stearylalkohol Verwendung finden.

Als Lösungsvermittler werden üblicherweise niedrige ein- oder mehrwertige Alkohole wie Ethanol, Isopropanol, Ethylenglykol, Propylenglykol, Glycerin, 1,3-Butylenglykol und Diethylenglykol verwendet.

Als Überfettungsmittel können Substanzen wie polyethoxylierte Lanolinderivate, Lecithinderivate und Fettsäurealkanolamide verwendet werden, wobei die letzteren gleichzeitig auch als Schaumstabilisatoren dienen.

Geeignete Verdickungsmittel sind beispielsweise Polysaccharide, insbesondere Xanthan-Gum, Guar-Gum, Agar-Agar, Alginate und Tylosen, sowie Carboxymethylcellulose und Hydroxyethylcellulose, ferner höhermolekulare Polyethylenglykolmono- und -diester von Fettsäuren, Polyacrylate, Polyvinylalkohol und Polyvinylpyrrolidon und schließlich Elektrolyte wie Kochsalz und Ammoniumchlorid, gewünschtenfalls in Kombination mit Alkylethersulfaten.

Unter biogenen Wirkstoffen sind Pflanzenextrakte, Eiweißabbauprodukte und Vitaminkomplexe zu verstehen.

Gebräuchliche Filmbildner sind beispielsweise Polyvinylpyrrolidon, Vinylpyrrolidon-Vinylacetat-Copolymerisate, Polymere der Acrylsäurereihe, quaternäre Cellulose-Derivate und ähnliche Verbindungen.

Als Konservierungsmittel eignen sich die in der Anlage zur Kosmetikverordnung aufgeführten Substanzen.

Als Perlglanzmittel kommen insbesondere Glykoldistearinsäureester wie Ethylenglykoldistearat, aber auch Fettsäuremonoglykolester in Betracht.

Als Farbstoffe können die für kosmetischen Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentrationen von 0,001 bis 0,1 Gew.-%, bezogen auf die gesamte Mischung, eingesetzt.

Weitere Komponenten der erfindungsgemäßen Mittel sind gewünschtenfalls Duftstoffe und Substanzen, die der Einstellung des pH-Wertes der Mittel dienen.

Die erfindungsgemäßen Mischungen werden bevorzugt in Produkten eingesetzt, die zum Waschen, Färben, Wellen oder Spülen von Haaren dienen. Insbesondere eignen sich die Mischungen für Shampoos zur Haarwäsche.

Die Erfindung wird durch die nachfolgenden Beispiele näher erläutert.

## Beispiele

### 1. Hautverträglichkeits-Untersuchungen

Zur Bestimmung der Hautverträglichkeit der Tensidmischungen wurde die von Zeidler und Reese entwickelte in-vitro-Methode verwendet, die in der Zeitschrift Ärztliche Kosmetologie 13, 39-45 (1983) ausführlich dargestellt ist.

Als Maß für die Hautverträglichkeit der Tensidmischungen diente die Quellung von Schweine-Epidermis. Dazu wurde die benötigte Epidermis unmittelbar nach der Schlachtung junger Schweine gewonnen und tiefgekühlt gelagert.

Für die Messung wurden ausgestanzte Epidermisstreifen der Größe 1 cm x 6 cm 30 Minuten lang in die Tensidlösungen getaucht, die jeweils 2 Gew.-% Aktivsubstanz enthielten, auf 39 °C temperiert und auf pH = 6,5 eingestellt waren. Sodann wurde nach kurzem Spülen und Entfernen des anhaftenden Wasser durch leichtes Abpressen unter definierten Bedingungen das Gewicht der gequollenen Streifen bestimmt. Anschließend wurden die Streifen 24 Stunden über Calciumchlorid entwässert und erneut gewogen.

Um Einflüsse auszuschalten, die auf spezifische Eigenschaften des jeweiligen Tieres oder den Entnahmeort (Rücken, Seite) zurückgehen, wurde jeweils eine Standardmessung durchgeführt. Dabei wird ein unmittelbar benachbarter Epidermisstreifen in gleicher Weise mit Wasser anstelle der Tensidlösung behandelt.

Die Meßwerte t für die Tensid-Behandlung und w für die Behandlung mit Wasser ergeben sich aus der Beziehung:

$$t, w = \frac{\text{Gewicht(gequollene Epidermis)} - \text{Gewicht(trockene Epidermis)}}{\text{Gewicht(trockene Epidermis)}}$$

4

Die standardisierte, relative Quellungsänderung Q ist schließlich definiert als

$$Q = (\frac{t}{w} - 1) * 100\%$$

Der Q-Wert der wasserbehandelten Haut ist somit definitionsgemäß 0 %; negative Werte weisen auf quellungshemmende Eigenschaften hin.

Es wurden die Eigenschaften von Mischungen einer Tensidmischung T mit Eucarol[R] TA[1] untersucht. Die Tensidmischung T wurde wie folgt hergestellt:

Ein Gemisch aus 96 kg eines Adduktes von 10 Mol Ethylenoxid an einem Kokosfettalkohol $C_{12}$-$C_{18}$ (54 Gewichtsprozent $C_{12}$, 22 Gewichtsprozent $C_{14}$, 10 Gewichtsprozent $C_{16}$, 12 Gewichtsprozent $C_{18}$), 113 kg eines Adduktes von 2 Mol Ethylenoxid an einem Kokosfettalkohol $C_{12}$-$C_{14}$ (73 Gewichtsprozent $C_{12}$, 27 Gewichtsprozent $C_{14}$) und 24,5 kg eines Adduktes von 2 Mol Ethylenoxid an einem Oleyl-Cetyl-Alkohol (65 Gewichtsprozent $C_{18}$, 30 Gewichtsprozent $C_{16}$, 5 Gewichtsprozent $C_{14}$, Jodzahl 50) wurde mit 74,4 kg Chlorsulfonsäure bei einer Temperatur von 10 bis 20 °C in einem kontinuierlichen Reaktor zum Schwefelsäurehalbester umgesetzt und anschließend mit 55,5 kg einer 50 %igen wässrigen Natriumhydroxidlösung, verdünnt mit 660 kg Wasser, neutralisiert. Das dabei erhaltene Alkylethersulfat hatte einen Aniontensid-Gehalt von 0,592 Mol/kg (bestimmt durch Zweiphasentitration nach DGF-Einheitsmethode H-III-10).

Zu 880 g dieses Ethersulfats wurden 28,2 g $MgSO_4 \cdot 7 H_2O$ (0,114 Mol), 4,6 g Trinatriumcitrat und 65 g Natriumchlorid (1,1 Mol) gegeben. Die so erhaltene Tensidmischung T hatte einen Aniontensid-Gehalt von ca. 0,59 Mol/kg entsprechend ca. 28 Gewichtsprozent (berechnet mit einem mittleren Molekulargewicht der Aniontenside von 475).

Für die Mischungen aus Tensidmischungen und Eucarol[R] TA[1] wurden folgende Q-Werte gemessen:

Mischungsverhältnis der Aktivsubstanzen

| Tensidmischung T : Eucarol TA | Q-Wert |
|---|---|
| 100 : 0 | 36 ± 11 |
| 60 : 40 | 16 ± 8 |
| 40 : 60 | 2 ± 7 |
| 20 : 80 | − 8 ± 6 |
| 0 : 100 | 36 ± 9 |

## 2. Bestimmung des Schaumverhaltens

Das Schaumverhalten der Tensidmischungen wurde mit einer motorisierten Schlag-Schaum-Apparatur in Anlehnung an DIN 53902 bestimmt.

Zur Bestimmung wurden 340 ml Tensidlösung (2 Gew.-% Aktivsubstanz in Leitungswasser aus Düsseldorf-Holthausen mit 18 °dH) hergestellt. Der Schaum wurde bei Raumtemperatur mit einer Lochplatte (Bohrungen von 1 mm Durchmesser, 10 Schläge bei einer Frequenz von 50 Schlägen/min, 13 cm Hub) erzeugt; er ist sehr feinporig und entspricht somit weitgehend einem beim Shampoonieren auf dem Kopf entstehenden Schaum. Die Messungen wurden ohne Fettbelastung der Tensidlösung als Doppelbestimmung durchgeführt.

Als Vergleich wurde das Schaumverhalten einer als gut schäumend bekannten Mischung aus
42 Gewichtsprozent Texapon[R] N 25[2] , (= 12 % Aktivsubstanz)
10 Gewichtsprozent Dehyton[R] K[3] ,(= 3 % Aktivsubstanz)
1 Gewichtsprozent Comperlan[R] LS[4] (= 1 % Aktivsubstanz) und

[1]Wäßrige Lösung von Natriumlaureth-7-tartrat, 25% Aktivsubstanz (ROL)
[2]Wäßrige Lösung von Natriumlaurylethersulfat (ca. 28 % Aktivsubstanz) (HENKEL)
[3]Wäßrige Lösung eines Fettsäureamid-Derivat mit Betainstruktur der Formel R-CONH-$(CH_2)_3$-$N_3(CH_+)_2$-$CH_2$-$COO_-$ mit der CFTA-Bezeichnung Cocoamidopropyl Betaine (ca. 30 % Aktivusubstanz, ca. 5 % NaCl, (HENKEL)
[4]Mischung von Kokosfettsäurediethanolamid (ca. 70 %) und Laurylalkohol + 2 Ethylenoxid (ca. 20 %); Rest: Wasser, freies Amin, freie Fettsäure, Ester (HENKEL)

47 Gewichtsprozent Wasser
bestimmt.

Diese Mischung wurde mit Wasser verdünnt und ebenfalls in Form einer Lösung mit 2 Gew.-% Aktivsubstanz eingesetzt. Es wurden folgende Schaummengen gemessen:

nach 1 Minute: 240 ml

nach 3 Minuten: 210 ml

nach 5 Minuten: 190 ml

Die gemessenen Schaummengen der erfindungsgemäßen Tensidlösungen sind in der folgenden Tabelle in Prozent bezogen auf die Schaummenge der Vergleichssubstanz angegeben.

Mischungsverhältnis
der Aktivsubstanzen                          relative Schaummenge
Tensidmischung T : Eucarol TA

| | | | nach 1 min | nach 3 min | nach 5 min |
|---|---|---|---|---|---|
| 100 | : | 0 | 87 | 95 | 95 |
| 60 | : | 40 | 71 | 71 | 79 |
| 40 | : | 60 | 88 | 90 | 95 |
| 20 | : | 80 | 83 | 81 | 84 |
| 0 | : | 100 | 88 | 90 | 84 |

3. Hautverträglichkeit von Mischungen mit ampholytischen/zwitterionischen Tensiden

Es wurden wäßrige Systeme untersucht, die 1,6 Gew.-% an Aktivsubstanz einer Mischung aus Tensidmischung T und Eucarol TA (20 : 80, bezogen auf die Aktivsubstanzen), 0,4 Gew.-% an Aktivsubstanz des ampholytischen/zwitterionischen Tensids A enthielten. In der Tabelle ist auch der Wert für eine Mischung, die 2 Gew.-% an Aktivsubstanz der binären Mischung aus Tensidmischung T und Eucarol TA enthält, angegeben.

Es wurden folgende Q-Werte gemessen:

| Tensid A | Q-Wert |
|---|---|
| – | $-8 \pm 6$ |
| Dehyton(R) AB 30[5] | $11 \pm 5$ |
| Dehyton(R) G[6] | $17 \pm 8$ |
| Dehyton(R) G-SF[7] | $0 \pm 7$ |
| Dehyton(R) CB[8] | $3 \pm 7$ |

[5] Wäßrige Lösung eines Fettamin-Derivates mit Betainstruktur, CTFA-Bezeichnung: Coco-Betaine (ca. 30 % Aktivsubstanz, ca. 6 % NaCl) (HENKEL)

[6] Wäßrige Lösung von N-Hydroxyethyl-N-Kokoalkylamidoethyl-glycinat-Natriumsalz, CTFA-Bezeichnung Cocoamphodiacetate, (ca. 30 % Aktivsubstanz, ca. 7 % NaCl) (HENKEL)

[7] Wäßrige Lösung von N-Hydroxyethyl-N-Alkylamidoethyl-propionat, CTFA-Bezeichnung Cocoamphodipropionate, (ca. 40 % Aktivsubstanz) (HENKEL)

[8] Wäßrige Lösung eines Fettamin-Derivates mit Betainstruktur, CTFA-Bezeichnung: Coco-Betaine (ca. 31 % Aktivsubstanz, ca. 6,5 % NaCl) (HENKEL)

Bei der Untersuchung analoger Systeme mit einer Mischung von Tensidmischung T und Eucarol TA (40 : 60, bezogen auf die Aktivsubstanzen) wurden folgende Q-Werte gemessen:

7

| Tensid A | Q-Wert |
|---|---|
| - | 2 ± 7 |
| Dehyton(R) AB 30 | - 4 ± 5 |
| Dehyton(R) G | - 3 ± 4 |
| Dehyton(R) G-SF | 1 ± 7 |
| Dehyton(R) CB | 6 ± 4 |
| Dehyton(R) K | 8 ± 5 |

Das Schaumverhalten dieser ternären Tensidmischungen wurde in gleicher Weise wie bei den binären Mischungen bestimmt. Die wäßrigen Mischungen enthielten 1,6 Gew.-% an Aktivsubstanz der binären Mischung Tensid T / Eucarol TA in dem unten angegebenen Verhältnis und 0,4 Gew.- % an Aktivsubstanz der dritten Tensidkomponente A.

Die gemessenen Schaummengen der erfindungsgemäßen Tensidlösungen sind in der folgenden Tabelle in Prozent bezogen auf die Schaummenge der Vergleichssubstanz angegeben. In der Tabelle ist auch der Wert für eine Mischung, die 2 Gew.-% an Aktivsubstanz der binären Mischung aus Tensidmischung T und Eucarol TA enthält, angegeben.

a) binäre Mischung Tensid T : Eucarol TA (40 : 60 bezogen auf Aktivsubstanz)

| Tensidkomponente A | relative Schaummenge | | |
|---|---|---|---|
| | nach 1 min | nach 3 min | nach 5 min |
| - | 88 | 90 | 95 |
| Dehyton K | 74 | 71 | 78 |
| Dehyton AB 30 | 83 | 76 | 89 |
| Dehyton G | 87 | 86 | 94 |
| Dehyton G-SF | 83 | 90 | 95 |
| Dehyton CB | 61 | 62 | 67 |

b) binäre Mischung Tensid T : Eucarol TA (20 : 80 bezogen auf Aktivsubstanz)

| Tensidkomponente A | relative Schaummenge | | |
|---|---|---|---|
| | nach 1 min | nach 3 min | nach 5 min |
| - | 88 | 90 | 95 |
| Dehyton K | 87 | 91 | 94 |
| Dehyton AB 30 | 83 | 81 | 94 |
| Dehyton G | 74 | 76 | 83 |
| Dehyton G-SF | 83 | 86 | 89 |
| Dehyton CB | 100 | 100 | 94 |

4. Rezepturbeispiele

a) Gut schäumendes mildes Shampoo:

| Komponente | Gewichtsteile |
|---|---|
| Eucarol[(R)] TA | 28,8 |
| Tensidmischung T | 17,1 |
| Dehyton[(R)] G-SF | 9,7 |
| Edenor[(R)] KPK[9] | 1,0 |
| Comperlan[(R)] KD[10] | 1,0 |
| Kochsalz | 2,0 |
| Wasser | ad 100 |

9 Kokospalmkernölfettsäure + 9,2 Ethylenoxid (HENKEL)

10 Kokosfettsäurediethanolamid (ca. 90 Aktivsubstanz)

Zusammensetzung der Fettsäure: ca. 56 % Laurinsäure

ca. 21 % Myristinsäure

ca. 10 % Palmitinsäure

ca. 12 % Stearin- und

Ölsäure

b) Klares farbloses Shampoo:

| Komponente | Gewichtsteile |
|---|---|
| Eucarol(R) TA | 28,8 |
| Tensidmischung T | 17,1 |
| Dehyton(R) G-SF | 9,7 |
| Comperlan(R) KD | 1,0 |

| | |
|---|---|
| Aminoxid WS(R) 35[11] | 1,0 |
| Antil(R) 141[12] | 1,0 |
| Kochsalz | 1,0 |
| Wasser | ad 100 |

[11] Wäßrige Lösung von Dimethylkokosacylamidopropylaminoxid (ca. 35 % Aktivsubstanz) (GOLDSCHMIDT)

[12] Wäßrige Lösung von Polyoxyethylen-propylenglykol-dioleat (ca. 40 % Aktivsubstanz) GOLDSCHMIDT)

c) Avivierendes Shampoo:

| Komponente | Gewichtsteile |
|---|---|
| Eucarol(R) TA | 28,8 |
| Tensidmischung T | 17,1 |
| Dehyton(R) G-SF | 9,7 |
| Edenor(R) KPK | 1,0 |
| Polymer JR(R) 400[13] | 1,0 |
| Kochsalz | 1,0 |
| Wasser | ad 100 |

[13] Hydroxyethylcellulose, quaterniert (UNION-CARBIDE)

d) farbloses, klares, mildes, gut schäumendes Shampoo mit cremigem Schaum

| Komponente | Gewichtsteile |
|---|---|
| Eucarol (R) TA | 28,8 |
| Tensidmischung T | 18,2 |
| Dehyton CB | 9,7 |
| Alkylglucosid[14] | 3,3 |
| Edenor(R) KPK | 1,0 |
| Kochsalz | 0,5 |
| Glucamate DOE-120[15] | 0,5 |
| Wasser | ad 100 |

[14] $R^1O(Z)_x$ mit Z = Glucose, x = 1,4 und $R^1$ = n-Alkyl($C_{12-14}$), 60,8 % Aktivsubstanz

[15] Poly(120)ethylenglykol-methylglucose-dioleat (AMERCHOL)

e) klares, mildes, gut schäumendes Avivageshampoo

| Komponente | Gewichtsteile |
|---|---|
| Eucarol(R) TA | 28,8 |
| Tensidmischung T | 18,2 |
| Dehyton(R) G-SF | 7,5 |
| Alkylglucosid[14] | 3,3 |
| Comperlan KD | 1,0 |
| Aminoxid WS(R) 35 | 1,0 |

```
Dehydol LS3[16]                          1,0

Kochsalz                                 1,0

Antil(R) 141                             1,0

Merquat(R) 550[17]                       1,5

Ethanol                                  2,0

Wasser                          ad 100
```

[16] $C_{12-14}$-Fettalkohol + 3 Ethylenoxid (HENKEL)

[17] Dimethyldiallylammoniumchlorid-Acrylamid-Copolymer, 8 % Aktivsubstanz in Wasser (MERCK)


## Patentansprüche

1. Oberflächenaktive wäßrige Mischungen enthaltend Fettalkoholethersulfate sowie mindestens ein weiteres Aniontensid aus der Gruppe der Fettalkoholethertartrate und Fettalkoholethercitrate, dadurch gekennzeichnet, daß (a) die Mischungen 0,01-3 Gew.-% an Erdalkalimetall-Ionen, bezogen auf die gesamte Mischung, enthalten und (b) die Fettalkoholethersulfate und die genannten weiteren Aniontenside in einem Mengenverhältnis von 70:30 bis 1:99 vorliegen.

2. Mischungen nach Anspruch 1, dadurch gekennzeichnet, daß sie 0,05-1 Gew.-%, insbesondere 0,05-0,5 Gew.-% an Erdalkalimetall-Ionen enthalten.

3. Mischungen nach einem der Ansprüche 1 oder 2, dadurch gekennzeichnet, daß sie als Erdalkalimetall-Ionen Magnesiumionen enthalten.

4. Mischungen nach einem der Ansprüche 1 bis 3, dadurch gekennzeichnet, daß sie Aniontenside in Mengen von 2-50 Gew.-%, insbesondere von 5-30 Gew.-%, bezogen auf die gesamte Mischung, enthalten.

5. Mischungen nach einem der Ansprüche 1 bis 4, dadurch gekennzeichnet, daß sie Fettalkoholethersulfate, -tartrate und/oder -citrate enthalten, deren Fettalkoholalkoxylatgruppen unabhängig voneinander einen Alkoxylierungsgrad von jeweils 2-15, insbesondere mit 3-10, aufweisen.

6. Mischungen nach einem der Ansprüche 1 bis 5, dadurch gekennzeichnet, daß sie Fettalkoholethersulfate und -tartrate mit einem Alkyl- oder Alkenylrest und/oder Fettalkoholethercitrate mit einem oder zwei Alkyl- oder Alkenylresten enthalten, die unabhängig voneinander linear oder verzweigt sind und 8-22 C-Atomen enthalten.

7. Mischungen nach einem der Ansprüche 1 bis 6, dadurch gekennzeichnet, daß sie eine Kombination aus Fettalkoholethersulfaten und Fettalkoholethertartraten enthalten.

8. Mischungen nach Anspruch 7, dadurch gekennzeichnet, daß sie als Fettalkoholethertartrat Natriumlauryl-(7 EO)-tartrat enthalten.

9. Mischungen nach einem der Ansprüche 1 bis 8, dadurch gekennzeichnet, daß sie zusätzlich 0,5-20 Gew.-%, insbesondere 1-10 Gew.-%, an ampholytischen und/oder zwitterionischen Tensiden, jeweils bezogen auf die gesamte Mischung, enthalten.

10. Mischungen nach einem der Ansprüche 1 bis 9, dadurch gekennzeichnet, daß sie einen pH-Wert von 5,5-

12

7,5, insbesondere von 6,5-7,5 aufweisen.

11. Mischungen nach einem der Ansprüche 1 bis 10, dadurch gekennzeichnet, daß sie als Shampoo formuliert sind.

12. Verwendung von Mischungen nach einem der Ansprüche 1 bis 11 zur Haarbehandlung.


**Claims**

1. Surface-active aqueous mixtures containing fatty alcohol ether sulfates and at least one other anionic surfactant from the group of fatty alcohol ether tartrates and fatty alcohol ether citrates, characterized in that (a) the mixtures contain 0.01 to 3% by weight alkaline earth metal ions, based on the mixture as a whole, and (b) the fatty alcohol ether sulfates and the other anionic surfactants mentioned are present in a quantitative ratio of 70 : 30 to 1 : 99.

2. Mixtures as claimed in claim 1, characterized in that they contain from 0.05 to 1% by weight and more preferably from 0.05 to 0.5% by weight alkaline earth metal ions.

3. Mixtures as claimed in claim 1 or 2, characterized in that they contain magnesium ions as alkaline earth metal ions.

4. Mixtures as claimed in any of claims 1 to 3, characterized in that they contain anionic surfactants in quantities of from 2 to 50% by weight and more especially 5 to 30% by weight, based on the mixture as a whole.

5. Mixtures as claimed in any of claims 1 to 4, characterized in that they contain fatty alcohol ether sulfates, tartrates and/or citrates of which the fatty alcohol alkoxxylate groups independently of one another have a degree of alkoxylation of 2 to 15 and more especially 3 to 10.

6. Mixtures as claimed in any of claims 1 to 5, characterized in that they contain fatty alcohol ether sulfates and tartrates containing an alkyl or alkenyl group and/or fatty alkyl ether citrates containing one or two alkyl or alkenyl groups which, independently of one another, are linear or branched and contain 8 to 22 C atoms.

7. Mixtures as claimed in any of claims 1 to 6, characterized in that they contain a combination of fatty alcohol ether sulfates and fatty alcohol ether tartrates.

8. Mixtures as claimed in claim 7, characterized in that they contain sodium lauryl (7 EO) tartrate as the fatty alcohol ether tartrate.

9. Mixtures as claimed in any of claims 1 to 8, characterized in that they additionally contain 0.5 to 20% by weight and more especially 1 to 10% by weight ampholytic and/or zwitterionic surfactants, based in either case on the mixture as a whole.

10. Mixtures as claimed in any of claims 1 to 9, characterized in that they have a pH value in the range from 5.5 to 7.5 and more especially in the range from 6.5 to 7.5.

11. Mixtures as claimed in any of claims 1 to 10, characterized in that they are formulated as a shampoo.

12. The use of the mixtures claimed in any of claims 1 to 11 for the treatment of hair.


**Revendications**

1. Mélanges tensioactifs aqueux renfermant des éthersulfates d'alcools gras ainsi qu'au moins un autre agent de surface anionique faisant partie du groupe des éthertartrates et des éthercitrates d'alcools gras, caractérisés en ce que (a) lesdits mélanges renfermant 0,01 à 3 % en poids d'ions de métaux alcalino-terreux par rapport à la totalité du mélange et en ce que (b) les éthersulfates d'alcools gras et les autres tensioactifs anioniques cités sont présents dans un rapport quantitatif de 70:30 à 1:99.

2. Mélanges selon la revendication 1, caractérisés en ce qu'ils renferment 0,05 à 1 % en poids, en particulier 0,05 à 0,5 % en poids, d'ions de métaux alcalino-terreux.

3. Mélanges selon l'une des revendications 1 ou 2, caractérisés en ce qu'ils renferment comme ions de métaux alcalino-terreux, des ions magnésium.

4. Mélanges selon l'une des revendications 1 à 3, caractérisés en ce qu'ils renferment des tensioactifs anioniques dans des proportions de 2 à 50 % en poids, en particulier de 5 à 30 % en poids, par rapport à l'ensemble du mélange.

5. Mélanges selon l'une des revendications 1 à 4, caractérisés en ce qu'ils renferment des éthersulfates, éthertartrates et/ou éthercitrates d'alcools gras, dont les groupes d'alcoxylates d'alcools gras présentent indépendamment l'un de l'autre un degré d'alcoxylation de 2 à 15, en particulier de 3 à 10 dans chaque cas.

6. Mélanges selon l'une des revendications 1 à 5, caractérisés en ce qu'ils renferment des éthersulfates et des éthertartrates d'alcools gras avec un radical alkyle ou alcényle et/ou des éthercitrates avec un ou deux radicaux alkyle ou alcényle, qui sont indépendamment l'un de l'autre linéaires ou ramifiés et comportent 8 à 22 atomes de C.

7. Mélanges selon l'une des revendications 1 à 6, caractérisés en ce qu'ils renferment une association d'éthersulfates d'alcools gras et d'éthertartrates d'alcools gras.

8. Mélanges selon la revendication 7, caractérisés en ce qu'ils renferment comme éthertartrate d'alcools gras, du lauryl-(7 EO)-tartrate sodique.

9. Mélanges selon l'une des revendications 1 à 8, caractérisés en ce qu'ils renferment en plus 0,5 à 20 % en poids, en particulier 1 à 10 % en poids, de tensioactifs ampholytes et/ou zwitterioniques, dans chaque cas par rapport à l'ensemble du mélange.

10. Mélanges selon l'une des revendications 1 à 9, caractérisés en ce qu'ils présentent un pH compris dans la plage de 5,5 à 7,5, en particulier de 6,5 à 7,5.

11. Mélanges selon l'une des revendications 1 à 10, caractérisés en ce qu'ils sont formulés en shampooing.

12. Mélanges selon l'une des revendications 1 à 11 pour le traitement des cheveux.